# EUROPÄISCHE PATENTSCHRIFT

(11) **EP 0 922 959 B1**
(45) Veröffentlichungstag und Bekanntmachung des Hinweises auf die Patenterteilung: **25.10.2006**
(21) Anmeldenummer: 98123340.6
(22) Anmeldetag: 08.12.1998
(51) Int. Cl.: G01N 35/00

(54) **Analysensystem für Probenflüssigkeiten**
Analysis system for liquid samples
Système d'analyse des échantillons liquides

(30) Priorität: 13.12.1997 DE 19755529
(43) Veröffentlichungstag der Anmeldung: 16.06.1999
(73) Patentinhaber: Roche Diagnostics GmbH, 68305 Mannheim (DE)
(72) Erfinder: Kintzig, Hans, 67311 Tiefenthal (DE)

(56) Entgegenhaltungen:
- EP-A- 0 377 503
- EP-A- 0 513 618
- EP-A- 0 794 424
- US-A- 4 118 280
- US-A- 5 314 661
- US-A- 5 447 690
- US-A- 5 686 047
- US-A- 5 690 892

## Beschreibung

Die Erfindung betrifft ein System zur Analyse von Probenflüssigkeiten beinhaltend Testelemente, ein feuchtigkeitsundurchlässiges, dicht abschließbares Vorratsbehältnis für mindestens 2 Testelemente und ein stromnetzunabhängiges Meßgerät. Die Erfindung betrifft weiterhin ein Verfahren zur Bestimmung eines Analyten in einer Probenflüssigkeit.

Analysenelemente, die es gestatten, einzelne Parameter in Probenflüssigkeiten zu bestimmen, sind seit geraumer Zeit bekannt und in einer großen Vielfalt kommerziell erhältlich. Insbesondere für den Bereich der medizinischen Diagnostik und der Umweltanalytik werden Systeme angeboten, die auch von wenig geschulten Personen bedient werden können. Einfach zu bedienen sind solche Systeme, die ohne die Verwendung von flüssigen Reagenzien arbeiten und bei denen im Allgemeinen keine oder zumindest keine aufwendige Vorbereitung des Probenmaterials notwendig ist. Für solche sogenannte Schnelltests wurde eine "Trockenchemie" entwickelt, bei der als Lösungsmittel allein das in der Probenflüssigkeit enthaltene Wasser dient.

Für die Bestimmung von Glukose in Blut sind beispielsweise Systeme gebrauchlich, bei denen der Patient geringe Mengen frisch gewonnenen Blutes auf eine Teststreifen aufgibt und die Messung mit einem einfach zu bedienenden Gerät, beispielsweise einem Reflexionsphotometer, durchführt. Bei den im Stand der Technik gebräuchlichsten Analysensystemen liegen die Testelemente separat außerhalb des Meßgerätes vor und werden erst zum eigentlichen Meßvorgang in das Meßgerät eingeführt.

Herkömmliche Meßgeräte besitzen eine Öffnung, beispielsweise einen Schlitz, in den ein Teststreifen von Hand eingeschoben werden kann. Führungselemente stellen sicher, daß ein Teststreifen in der vorgesehenen Orientierung eingeschoben wird. Um die gewünschte Positionierung des Teststreifens zu gewährleisten müssen konstruktive Merkmale des Gerätes vorliegen. Üblicherweise wird dies durch eine Begrenzung realisiert, die ein Einschieben über eine vorgegebene Zielposition verhindert.

Obwohl in letzter Zeit zunehmend Systeme basierend auf elektrochemischen Sensoren zum Kauf angeboten werden, verwenden die gebräuchlichsten Systeme analytische Tests, die auf Farbänderungen beruhen, die sich im Verlauf der Nachweisreaktion bei Anwesenheit der zu bestimmenden Analyten zeigen. Die Detektion der auf dem Teststreifen aufgetretenen Farbänderung kann reflexionsphotometrisch erfolgen. Eine Messung in Transmission ist ebenfalls möglich, setzt jedoch zumindest teilweise transparente Teststreifen voraus. Die für die Photometrie notwendigen Vorrichtungen zur Erzeugung und Detektion von Strahlung sind prinzipiell im Stand der Technik bekannt.

Teststreifen des Standes der Technik besitzen ein Testfeld und eine Halterung dieses Testfeldes, meist in Form einer steifen Folie, die eine bequeme und sichere Handhabung des Teststreifen ermöglicht. Das Testfeld seinerseits kann aus mehreren Schichten bestehen. Für Bestimmungen von Analyten im Blut sind zum Beispiel Schichten aus Vliesmaterialien üblich, die eine Abtrennung zellulärer Blutbestandteile vom Serum bewirken. Außerdem sind Schichten gebräuchlich, in denen Reaktionen ablaufen oder die zur Dosierung des Probenmaterials beitragen. Typische Aufbauten von Teststreifen sind beispielsweise in den deutschen Patentanmeldungen DE 196 296 56.0 und DE 196 296 57.9 sowie in EP-B 0 271 854, EP-A-0 487 068, WO 92/17768 und DE-A 195 23049 beschrieben.

Der Nachweis eines Analyten erfolgt nur in wenigen Fällen durch Reaktion des Analyten mit einer einzigen Substanz und die direkte Bildung eines Farbstoffes. In der Regel läuft eine Kette von chemischen und/oder biochemischen Reaktionen ab, die schließlich zu einer beobachtbaren Farbveränderung führen. Das Testfeld, auf dem die Farbreaktion zu beobachten ist, muß in Größe und Formgebung so gestaltet sein, daß gewährleistet ist, daß auch bei Fertigungstoleranzen von Meßgerät und Testelement ein gleichbleibend großes Feld be- bzw. durchstrahlt werden kann.

Aus dem Stand der Technik bekannte Testelemente besitzen im wesentlichen die Form eines flachen Streifens oder einer flachen, rechteckigen Platte. Als Materialien sind Papier, spezielle Pappen und Kunststoffe gebräuchlich. Das Testfeld ist entweder durch Imprägnierung des Materials mit entsprechenden Reagenzien oder in Form von zusätzlichen Schichten auf die genannten Materialien aufgebracht.

Die Lagerungsbeständigkeit vieler Testelemente wird durch den Einfluß von Feuchtigkeit, beispielsweise aus der Umgebungsluft, stark herabgesetzt, da die Testfelder oftmals empfindliche, meist biochemische Reagenzien enthalten. Aus diesem Grund sind im Handel erhältliche Testelemente entweder einzeln eingesiegelt oder werden in größeren Mengen in speziellen Behältnissen verpackt zur Verfügung gestellt. Eine Versiegelung erfolgt in aller Regel in kunstofflaminierten Metallfolien, bevorzugt Aluminiumlaminaten. Diese Verpackungsform findet sich vor allem bei Schnelltests, die apparativ oder visuell ausgewertet werden. Zur Verwendung eines eingesiegelten Testelements wird die Verpackung manuell aufgerissen und das Testelement entnomnen. Teststreifen, die von einem Benutzer häufiger verwendet werden, beispielsweise Blutglukoseteststreifen für Diabetiker, befinden sich im Allgemeinen zu mehreren in wiederverschließbaren Behältnissen aus feuchtigkeitsundurchlässigen Materialien, die zusätzlich noch Trockenmittel enthalten können, um eingetretene Feuchtigkeit zu absorbieren.

Der Meßvorgang mit den bekannten Systemen zur Analyse mit trockenchemischen Schnelltests erfolgt unter Verwendung eines Analysengerätes und separater Teststreifen zum einmaligen Gebrauch. Der Anwender des Systems öffnet dazu manuell ein Vorratsbehältnis, entnimmt einen Teststreifen und verschließt das Behältnis wieder, um im Vorratsbehältnis verbliebene Testreifen vor Feuchtigkeit zu schützen. Nachfolgend wird die zu analysierende Probenflüssigkeit auf die Probenaufgabezone des Teststreifens gegeben. Der Teststreifen wird entweder direkt nach der Probenaufgabe oder nach dem Verstreichen einer Inkubationszeit in das Analysengerät eingeführt. Bei neueren Systemen erfolgt die Probenaufgabe erst, wenn sich der Teststreifen im Gerät befindet. Bei beiden Vorgehensweisen findet das Einschieben des Teststreifens im Meßgerät manuell durch den Anwender statt. Das Positionieren des Teststreifens wird durch konstruktive Maßnahmen gewährleistet.

Der eigentliche Meßprozeß wird in der Regel durch einen Tastendruck des Bedieners ausgelöst. Es existieren auch Systeme, bei denen der Meßvorgang durch das Einschieben des Teststreifens in das Gerät automatisch gestartet wird. Nach dem Abschluß der Messung, der für den Anwender aufgrund einer Anzeige am Gerät zu erkennen ist, wird der Analysenprozeß durch die Entnahme des Teststreifens beendet.

Nachteilig an den beschriebenen Systemen zur Analyse von Probenflüssigkeiten ist, daß der Anwender in der Regel mehrere Handhabungsschritte mit den Testelementen durchführen muß. Da die Benutzer solcher Systeme gerade im Bereich der medizinischen Diagnostik oftmals Diabetiker sind, denen eine sichere Handhabung kleiner Testelemente bisweilen große Probleme bereitet, stellt sich die Aufgabe, Analysensysteme so zu konstruieren, daß eine Handhabung von einzelnen Testelementen entfällt oder auf ein Mindestmaß reduziert ist.

Ein weiterer Nachteil bestehender Systeme liegt in der manuellen Positionierung der Teststreifen durch den Anwender, die eine Fehlbenutzung nicht zuverlässig verhindert. Demgemäß wird angestrebt, Systeme zur Verfügung zu stellen, mit denen eine Positionierung der Testelemente so erfolgt, daß Bedienungsfehler, die auf unsachgemäßer Positionierung beruhen, zuverlässig vermieden werden.

US 4,118,280 beschreibt ein automatisiertes Analysegerät, in dem ein Kartenstapel in einem kassettenförmigen Vorratsbehältnis angeordnet ist. Die Karten werden seitlich über Nuten in dem Behälter geführt und von einem Mechanismus aus dem Behälter entnommen bzw. dort eingelegt. In EP 0 277 503 ist ein Vorratsbehältnis für Testelemente beschrieben, das durch eine asymmetrische Anordnung sicherstellt, dass die Testelemente nur in der korrekten Orientierung eingesetzt werden können. US 5,314,661 betrifft ebenfalls ein Vorratsbehältnis für analytische Testelemente. Hierbei werden die Testelemente übereinander gestapelt gelagert und zur Prozessierung einzeln entnommen.

Da die Teststreifen, die bei den üblichen Systemen verwendet werden, im Allgemeinen produktionsbedingte Schwankungen in ihrer Nachweischarakteristik zeigen, ist es notwendig, chargenspezifische Kenndaten für eine korrekte Auswertung an das Meßgerät zu übermitteln.

Dazu werden heutzutage oftmals Chargencodes verwendet, die entweder direkt auf die Teststreifen aufgebracht sind und beim Einführen der Streifen in das Gerät automatisch erfaßt werden, oder die sich auf der Verpackung der Teststreifen befinden und manuell vom Benutzer, beispielsweise mittels einer Tastatur, eingegeben werden müssen. Beide Varianten stellen keine optimale Lösung dar. Zur Codierung der einzelnen Teststreifen ist vom Hersteller zunächst eine stichprobenartige Analyse der Funktionscharakteristik erforderlich, bevor die Streifen mit einem Code versehen werden können. Der Produktionsprozeß kann somit nicht in einem Schritt abgeschlossen werden, was den Prozeß umständlich und damit letztendlich teuer macht. Wird hingegen die Verpackung mit dem Code versehen, kann der Produktionsprozeß der Streifen zwar vor der Codierung abgeschlossen werden, bei der manuellen Übermittlung des Codes von der Verpackung an das Gerät sind jedoch Fehler möglich, beispielsweise falsche oder fehlende Eingabe durch den Benutzer. Eine Optimierung der Codeübermittlung wird deshalb ebenfalls angestrebt.

Die Aufgabe der vorliegenden Erfindung bestand darin, die Nachteile des Standes der Technik zu beseitigen.

Dies wird durch den Gegenstand der Erfindung, wie er in den Patentansprüchen charakterisiert ist, erreicht.

Gegenstand der Erfindung ist ein System zur Analyse von Probenflüssigkeiten beinhaltend
a) Testelemente mit Randprofil,
b) ein feuchtigkeitsundurchlässiges, dicht abschließbares Vorratsbehältnis für mindestens 2 Testelemente enthaltend ein Führungselement, einen Abstandhalter zwischen Führungszapfen und dem als ersten zu entnehmenden Testelement, und eine Transportvorrichtung für Testelemente
c) ein stromnetzunabhängiges, mit einer Hand haltbares Meßgerät enthaltend eine Führungsnut für Testelemente, einer gegenüberliegenden Führungsnut für das Führungselement des Testelementevorratsbehättnisses, sowie eine Haltevorrichtung für Testelemente.

Bei dem erfindungsgemäßen System handelt es sich um ein Meßgerat mit einem separaten, das heißt, nicht dauerhaft mit dem Meßgerät verbundenen, d. h. abnehmbaren Vorratsbehältnis, z.B. in Form einer Spenderpackung für Testelemente. Das Meßgerät ist vorzugsweise so dimensioniert, daß es bequem vom Anwender in einer Hand gehalten werden kann. Die Spannungsversorgung erfolgt aus praktischen Gründen stromnetzunabhängig, vorzugsweise mit Batterien, Akkumulatoren oder Solarzellen. Das Meßgerät verfügt über eine gut ablesbare Anzeige für die Meßwerte, vorzugsweise in Form eines Flüssigkristall-Displays (LCD).

Das Meßgerät besitzt vorzugsweise im wesentlichen die Form eines flachen, länglichen Quaders. Das Meßgerätegehäuse ist aus einem leichten und stabilen Werkstoff, vorzugsweise einem Leichtmetall oder einem Kunststoff gefertigt. Auf der Außenseite des Gerätegehäuses können sich je nach gewünschtem Bedienkomfort erforderliche Schalter befinden, beispielsweise Schalter zum Ein- und Ausschalten des Gerätes sowie gegebenenfalls Bedienungsknöpfe für weitere Funktionen, wie Uhrzeitanzeige, Speicherung der Meßwerte etc. In einer besonders vorteilhaften Ausführungsform weist das Gerät einen Druckschalter zur Betätigung eines Auswerfmechanismus für im Gerät befindliche Testelemente auf. Auf der Vorderseite des Gerätes befindet sich die Meßwertanzeige, vorzugsweise in Form eines Flüssigkristall-Displays sowie in einer besonders bevorzugten Ausführungsform eine Führungsnut für Testelemente, in der eine Haltevorrichtung für Testelemente integriert ist. Der Führungsnut für Testelemente gegenüber, vorzugsweise auf der Rückseite des Meßgerätegehäuses, befindet sich eine Aussparung, die als Führungsnut für das Führungselement des Vorratsbehältnisses für Testelemente ausgestaltet ist. Alternativ dazu können die Führungsnuten auch nebeneinander angeordnet sein, wobei sowohl Vorder- und Rückseite des Gerätes die Nuten enthalten können. Weiterhin kann ein Batterie- oder Akkumulatorfach für die Spannungsversorgung des Meßgerätes vorgesehen sein, vorzugsweise auf der Rückseite. Vorzugsweise wird das Gerät automatisch eingeschaltet, beispielsweise durch das Einführen eines Teststreifens in das Gerät. Das Abschalten kann ebenfalls automatisch erfolgen, beispielsweise beim Entfernen des benutzten Teststreifens aus dem Gerät. Beide Funktionen können jedoch auch manuell, beispielsweise durch Bedienen eines Schalters oder Tasters, auszuführen sein. Möglich ist auch, daß sowohl manuelle als auch automatische Bedienung möglich bzw. erforderlich ist. Funktionale und konstruktive Details der erfindungswesentlichen Bestandteile des Meßgeräts werden weiter unten beschrieben.

Mit dem erfindungsgemäßen System ist die Entnahme der Testelemente aus der Packung und das Einführen in das Gerät gegenüber herkömmlichen Systemen wesentlich erleichtert. Die Testelemententnahme erfolgt direkt mit dem und durch das Gerät. Das bedeutet, daß die Testelemente nicht mehr vom Anwender von Hand eingelegt werden müssen. Eine Grifffläche am Testelement ist somit nicht mehr notwendig. Die Größe der Testelemente wird folglich nicht mehr wie bisher üblich von Handhabungskriterien bestimmt, sondern kann funktional bestimmt auf die minimal mögliche Größe reduziert werden. Dadurch wird beim Herstellen der Testelemente Material eingespart. Die geringere Größe wirkt sich auch in einer geringeren Packungsgröße bei gleicher Anzahl Testelemente positiv aus.

Das Vorratsbehältnis ist für mindestens 2 Testelemente ausgelegt. Praktischerweise liegt die Gesamtzahl der zu bevorratenden Testelemente zwischen 10 und 100 Stück, vorzugsweise bei 50 Stück. Bevorzugt ist das Vorratsbehältnis als Spenderpackung ausgeführt, bei der durch einen Mechanismus dafür gesorgt wird, daß an einer speziellen Entnahmeposition stets ein Testelement zur Entnahme bereit steht. Die Spenderpackung ist vorzugsweise aus einem möglichst feuchtigkeitsundurchlässigen Material, insbesondere einem im wesentlichen feuchtigkeitsundurchlässigen Kunststoff, gefertigt. Hierbei wird unter feuchtigkeitsundurchlässig verstanden, daß Wasser sowohl als Flüssigkeit als auch als Dampf nicht durch das Material der Spenderpackung dringen kann oder zumindest stark am Durchtritt gehindert wird. Bevorzugt ist die Spenderpackung mit einer Klappe ausgestattet und durch diese zu öffnen bzw. zu verschließen. Die Spenderpackung ist bei geschlossener Klappe beispielsweise durch Dichtlippen oder O-Ringe luftdicht abgedichtet, um ein Eindringen von Feuchtigkeit weitestgehend zu verhindern.Im Innern des Vorratsbehältnisses befindet sich vorzugsweise ein Trockenmittel, um Feuchtigkeit, die z. B. beim Öffnen des Behältnisses in das Vorratsbehältnis eindringen kann, zu absorbieren und dadurch die Testelemente vor Feuchtigkeit zu schützen

Unter der Klappe befindet sich bei einer bevorzugten Ausführungsform in geschlossenem Zustand das Führungselement, das beim Öffnen der Klappe zugänglich wird und über das das Gerät bei der Testelementeentnahme geschoben wird. In einem vorbestimmten, durch einen Abstandhalter definierten Abstand zum Führungselement befindet sich ein Testelement mit einem, vorzugsweise stufenförmigen, Randprofil in der Entnahmeposition. Alle weiteren Testelemente sind in der Spenderpackung, vorzugsweise sich paarweise mit ihren Flächen berührend, neben- bzw. hintereinander gestapelt und in einer besonders bevorzugten Ausführungsform an einer kleinen Kontaktstelle leicht und wieder lösbar miteinander verklebt, um eine gleichbleibende Orientierung zueinander, insbesondere beim Beladen des Vorratsbehältnisses mit den Testelementen, zu gewährleisten. Ein einmal entnommenes Testelement wird durch eine Transportvorrichtung selbständig, vorzugsweise durch einen Federmechanismus, durch das jeweils nachfolgende Testelement ersetzt. Die Innenwände des Vorratsbehältnisses sind vorzugsweise so geformt, daß sie als Führung für den Testelementetransport dienen.

Der Federmechanismus entspricht vorzugsweise im wesentlichen dem Transportmechanismus, wie er aus Gewehr- oder Pistolenmagazinen oder aus Heftklammerern bekannt ist. Er enthält als wesentliche Teile eine Spiralfeder und eine vorzugsweise durch Führungsschienen oder -stangen bzw. Wandprofile geführte Platte, die den Federdruck auf die Testelemente überträgt und somit für eine gerichtete Bewegung der Testelemente vom Behältnisinneren zur Entnahmeposition sorgt.

Das Testelementevorratsbehältnis ist vorzugsweise als flacher, stabförmiger Quader ausgeführt, der an einem Ende eine Klappe aufweist. Ganz besonders bevorzugt befindet sich unter der Klappe ein Testelement in Entnahmeposition, ein Führungselement sowie ein Abstandhalter, der das Führungselement und das Testelement in der Entnahmeposition auf Distanz hält.

In einer besonders bevorzugten Ausführungsform enthält das Testelementevorratsbehältnis außer der Entnahmeöffnung, die beispielsweise mit einer Klappe versehen ist, auch eine dicht verschließbare Öffnung zum Befüllen des Behältnisses mit Testelementen. Vorzugsweise ist das Testelementevorratsbehältnis mehrfach zu benutzen, wozu bei Bedarf die Testelemente vom Benutzer einfach nachgefüllt werden können müssen. Beispielsweise kann eine der Begrenzungsflächen des Vorratsbehältnisses reversibel entfernt werden, so daß die nachzufüllenden Testelemente einzeln oder bevorzugt miteinander, beispielsweise über eine lösbare Verklebung verbunden in das Behältnis eingeführt werden können. Besonders bevorzugt wird durch den Auffüllvorgang der Transportmechanismus - z. B. durch Spannen einer Feder - reaktiviert.

Vorzugsweise erfolgt das Beladen des Vorratsbehältnisses mit Testelementen vom Boden des Behälters her. Der Boden ist bei im wesentlichen quaderförmigen Behältnissen diejenige Begrenzungsfläche des Vorratsbehältnisses, die im wesentlichen senkrecht zur Transportrichtung der Testelemente im Behältnis liegt. In einer bevorzugten Ausführungsform ist der Boden über einen Rastmechanismus fest und dicht mit dem Behältniskörper zu verbinden. Zum Einlegen neuer Testelemente in das Vorratsbehältnis wird der Rastmechanismus gelöst und der Boden, an dem vorzugsweise der Transportmechanismus für die Testelemente, beispielsweise also eine Feder und eine oder mehrere Führungsschienen oder -stangen befestigt ist, vom Behältnis entfernt. Der Boden kann dabei wie eine Schublade über eine Führung mit dem Behältnis verbunden sein oder vollständig ablösbar vorliegen. Für den Fall, daß der Boden im wesentlichen vollständig ablösbar ist, können die nachzufüllenden Testelemente direkt in das Innere des Testelemenetvorratsbehältnisses gefüllt werden. Andernfalls können die Testelemente in oder auf die Führung, mit der der Boden wie eine Schublade mit dem Behältnis verbunden ist, gegeben und damit in das Innere des Behältnisses befördert werden. Das Reaktivieren des Transportmechanismus erfolgt beispielsweise durch Spannen der Feder(n) beim Einlegen der Testelemente, ähnlich dem Beladen von Heftklammerern mit Heftklammern. Die nachzufüllenden Testelemente können in einer Transportverpackung, beispielsweise einer dicht schließenden Dose, Röhre, verschweißten Folie oder einer Blisterpackung zur Verfügung gestellt werden, aus der sie zum Befüllen des Vorratsbehältnisses entnommen werden müssen.

Das Vorratsbehältnis kann alternativ zur Quaderform auch in Form einer flachen Scheibe, beispielsweise einer quadratischen oder runden Scheibe, ausgestaltet sein, die eine Aussparung am Rand aufweist, in die das Meßgerät zur Testelemententnahme eingeführt werden kann.

Das Führungselement des Vorratsbehältnisses sorgt in Verbindung mit der entsprechenden Führungsnut des Meßgerätes während des Entnahmevorgangs der Testelemente für eine eindeutige Orientierung des Meßgerätes zum Vorratsbehältnis und damit auch zu den darin angeordneten Testelementen. Eine falsche Orientierung wird durch entsprechende Dimensionierung von Führungsnut und Führungselement ausgeschlossen. Die Testelemente können somit nicht in einer falschen Ausrichtung eingeführt werden.

Vorzugsweise ist das Führungselement als Zapfen ausgestaltet, der sich ganz besonders bevorzugt in der Nähe des Randes des Vorratsbehältnisses befindet.

Der Führungszapfen ist in einer bevorzugten Ausführungsform im wesentlichen quaderförmig, wobei eine Grundfläche des Quaders mit dem Vorratsbehältnis fest verbunden ist. Die korrespondierende Führungsnut im Meßgerät ist eine in Form und Größe an den Führungszapfen angepaßte, ebenfalls im wesentlichen quaderförmige Vertiefung im Meßgerätegehäuse. Beim Zusammenführen von Testelementvorratsbehältnis und Meßgerät zum Zwecke der Testelemententnahme aus dem Vorratsbehältnis mit dem Meßgerät gleitet der Führungszapfen in der Führungsnut. Dadurch wird der Weg des Meßgeräts relativ zum Vorratsbehältnis bestimmt, womit gleichzeitig eine eindeutige Orientierung des Meßgeräts zu den im Vorratsbehältnis enthaltenen Testelementen garantiert ist.

Führungselement und Führungsnut sind vorzugsweise so aneinander angepaßt, daß ein Zusammenführen des Testelementvorratsbehältnisses und des Meßgerätes ohne große Kraftaufwendung möglich ist. Die Führungsnut ist dazu geringfügig größer gehalten als das Führungselement, ohne daß beide Komponenten zuviel Spiel aufweisen. Bevorzugt ist die Führungsnut auf der Rückseite, das heißt auf der der Meßwertanzeige gegenüberliegenden Seite des Meßgerätes angeordnet und weist eine leicht konische oder trichterförmige Öffnung auf, in die das vorzugsweise ebenfalls konische, Führungselement leicht eingeführt werden kann. Ebenfalls erleichternd für das Zusammenführen von Führungselement und -nut wirkt eine Abrundung der Kanten von Führungselement und -nut, wie sie weiterhin besonders bevorzugt ist.

In oder auf dem Führungselement befindet sich in einer bevorzugten Ausführungsform, bevorzugt auf der gerätezugewandten Seite, eine chargenspezifische Codierung. In der entsprechenden Führungsnut des Meßgerätes befindet sich eine entsprechende Codelesevorrichtung, die den Code beim Aus- oder Einschieben des Führungselements automatisch liest und dem Meßgerät zur Auswertung zur Verfügung stellt. Als Codierung kommen insbesondere optische oder elektromagnetische Codes in Frage, bevorzugt Strichcodes, Magnetstreifencodes oder allgemein Codes, die in ein sogenanntes "Read-Only-Memory" (ROM) eingespeichert sind. Das Meßgerät enthält jeweils entsprechende Vorrichtungen, die zum Lesen des Codes geeignet sind. Codierungs- und Auslesemöglichkeiten sind dem Fachmann in großer Vielzahl bekannt und sollen hier nicht näher erläutert werden.

Bei der Entnahme des Streifens wird der chargenspezifische Code der Testelemente automatisch zum Gerät übermittelt. Damit ist die verwechslungsfreie Übermittlung der Chargenkennung des jeweils untersuchten Testelements in allen Fällen sichergestellt. Vorzugsweise ist das Gerät vor der Testelemententnahme bereits eingeschaltet, so daß der Code bei der Testelemententnahme bereits beim Zusammenführen des Gerätes mit der Vorratspackung eingelesen wird. Alternativ dazu kann der Code beim Trennen von Gerät und Spenderpackung eingelesen werden, beispielsweise wenn das Einschalten des Gerätes automatisch durch das Einführen des Testelements in das Gerät erfolgt.

In einer bevorzugten Ausführungsform befindet sich am unteren Ende des Gerätes auf der Vorderseite eine Führungsnut für die Testelemente. Die Vorderseite des Geräts ist dabei die Seite, auf der sich die Meßwertanzeige, beispielsweise ein LCD-Display, befindet. Das untere Ende des Gerätes ist im Gegensatz zum oberen Ende des Gerätes dasjenige Ende, welches den größeren Abstand zur Meßwertanzeige hat. Diese Testelemente sind vorzugsweise entlang ihres Randes so profiliert, daß zumindest ein Teil des Randes eine Feder bildet, die in die entsprechende Führungsnut des Gerätes paßt. Vorzugsweise am gegenüberliegenden unteren Ende des Gerätes, das heißt beispielsweise auf dessen Rückseite, ist eine Führungsnut vorhanden, in die das Führungselement der Spenderpackung paßt. Bevorzugt sind die beiden Führungsnuten unterschiedlich dimensioniert, das heißt, sie besitzen unterschiedliche Größe und / oder unterschiedliche Gestalt, so daß ein irrtümliches falsches Einschieben des Gerätes ausgeschlossen wird. Damit ist zudem sichergestellt, daß die Testelemente automatisch richtig in das Gerät eingeführt werden.

Bei der Testelementeentnahme wird das Gerät mit dem unteren Ende voran bevorzugt von oben im rechten Winkel zur Packung zwischen Führungselement und Testelement geschoben, bis das profilierte Testelement in die dafür vorgesehene Führungsnut gleitet. Das Testelement wird durch eine Haltevorrichtung im Meßgerät in der optimalen Position gehalten. Das Erreichen der endgültigen Position wird dem Nutzer taktil und akustisch mitgeteilt. Das Auffinden der richtigen Position wird durch die rückwärtige Führungsnut des Gerätes in Verbindung mit dem Führungselement des Vorratsbehältnisses verwechslungsfrei sichergestellt. Beim Herausziehen des Gerätes wird der Teststreifen durch die Haltevorrichtung in der eingerasteten Position mitgeführt. Als Haltevorrichtung dient vorzugsweise ein Rastmechanismus, der in eine Aussparung, zum Beispiel ein Loch, des Testelements einrastet. Beispielsweise kann die Haltevorrichtung aus einer federgelagerten Kugel oder einem vorgespannten Kunststoffhaken oder einem der in EP-A 0 618 443 genannten Mechansimen bestehen. Zum Entfernen des Testelements nach erfolgter Analyse ist im Meßgerät in einer bevorzugten Ausfiihrungsform eine Auswerfvorrichtung vorgesehen, so daß der Benutzer des Systems das Testelement nach der Messung nicht mit den Händen anfassen muß, womit eine hygienische Entsorgung benutzter Testelemente gewährleistet ist. Beispielsweise kann als Auswerfmechanismus ein über einen Druckknopf zu betätigendes Schubgestänge dienen. Durch Drücken auf den Druckknopf wird das Testelement gegen den Widerstand des Haltemechanismus in Druckrichtung aus dem Gerät geschoben und schließlich ausgeworfen.

Da die Testelemente des erfindungsgemäßen Systems mit Hilfe des Meßgeräts direkt aus dem Vorratsbehältnis entnommen werden, findet die Probenaufgabe auf ein sich im Meßgerät befindliches Testelement statt. Um eine Kontamination des Meßgeräts weitestgehend zu verhindern, beispielsweise durch Blut, sind die Testelemente praktikablerweise so dimensioniert, daß sie nach dem Einbringen in das Meßgerät geringfügig, vorzugsweise 5 bis 15 mm, aus dem Meßgerät ragen. Die Probenaufgabe findet vorzugsweise auf das aus dem Meßgerät ragende Ende des Testelements statt. Da für kolorimetrische Teste praktischerweise die Detektionseinheit des Meßgerätes im Geräteinneren liegt, ist es bevorzugt, daß auch das Testfeld des Testelements zumindest teilweise im Innern des Gerätes zu liegen kommt, so daß eine Detektion überhaupt erst möglich ist. Dazu ist es erforderlich, daß die Probe durch geeignete, im Testelement enthaltene Mittel von der Probenaufgabestelle zur Detektionsstelle transportiert wird. Besonders bevorzugtes Mittel zum Transport der Probe von der Probenaufgabestelle zur Detektionsstelle ist ein kapillaraktiver Kanal, wie er beispielsweise aus EP-A 0 487 068, EP-A 0 215 419 oder DE-A 31 51 291 bekannt ist. Jedoch sind auch andere Mittel zum Tranport der Probe möglich, beispielsweise Vliese mit Dochtwirkung oder ähnliches.

Bei elektrochemischen Detektionsprinzipien, beispielsweise bei amperometrischen oder potentiometrischen Sensoren und Biosensoren, kann die Probenflüssigkeit direkt auf eine außerhalb des Meßgeräts gelegene Detektionsstelle aufgegeben werden. Zur Detektion des elektrischen Nachweissignals wird dieses mit Hilfe geeigneter Leitungselemente, beispielsweise gedruckter oder geäzter Leiterbahnen, in das Meßgerät geführt, dort gegebenenfalls verstärkt, analysiert und angezeigt.

Weiterhin ist Gegenstand der Erfindung ein Verfahren zur Bestimmung eines Analyten in einer Probenflüssigkeit mit Hilfe eines erfindungsgemäßen Systems. Das Verfahren zeichnet sich dadurch aus, daß zunächst aus dem geöffneten Vorratsbehältnis mit Hilfe des Meßgerätes ein Testelement entnommen wird. Auf das sich im Gerät befindliche Testelement wird auf die Probenauftragszone eine Probenflüssigkeit aufgebracht, welche selbständig in die Detektionszone gelangt, dort mit den Komponenten des Testfeldes eine charakteristische, detektierbare Reaktion, beispielsweise unter Farbveränderung oder von einer elektrochemisch beobachtbaren Umwandlung begleitet, eingeht. Das Ergebnis der Reaktion wird mit Hilfe des Gerätes erfaßt und angezeigt. Schließlich wird nach der Analyse das benutzte Testelement, gegebenenfalls mit Hilfe der Auswerfvorrichtung, aus dem Meßgerät entfernt.

Die Erfindung wird durch die Figuren 1 bis 7 erläutert.
Figur 1 zeigt eine besonders bevorzugte Ausführungsform des Meßgerätes des erfindungsgemäßen Systems in unterschiedlichen Ansichten (a) Frontansicht; b) Rückansicht: c) Seitenansicht; d) Ansicht von unten).
Figur 2 zeigt eine besonders bevorzugte Ausführungsform des Testelements mit Randprofil des erfindungsgemäßen Systems in unterschiedlichen Ansichten (a) von oben; b) von unten; c) Seitenansicht; d) von vorne auf die Stirnfläche).
Figur 3 ist eine besonders bevorzugte Ausführungsform des Testelementevorratsbehälters des erfindungsgemäßen Systems in geschlossenem (a) und geöffneten (b) Zustand.
Figur 4 zeigt schematisch, wie mit Hilfe des erfindungsgemäßen Systems ein Testelement durch das Meßgerät aus dem Vorratsbehältnis entnommen wird.
Figur 5 zeigt einen Längsschnitt durch eine besonders bevorzugte Ausführungsform des Testelementevorratsbehälters des erfindungsgemäßen Systems.
Figur 6 zeigt einen Längsschnitt von der Seite (a) und von oben (b) sowie einen Querschnitt (c) durch eine weitere besonders bevorzugte Ausführungsform des Testelementevorratsbehälters des erfindungsgemäßen Systems in geschlossenem Zustand.
Figur 7 zeigt einen Längsschnitt von der Seite (a) und von oben (b) durch die besonders bevorzugte Ausführungsform des Testelementevorratsbehälters des erfindungsgemäßen Systems gemäß Figur 6, wobei der Boden zum Beladen mit Testelementen geöffnet ist.

Die Ziffern in den Figuren bedeuten:
- 1: Meßgerätegehäuse
- 2: Display
- 3: Führungsnut für Testelement
- 4: Haltevorrichtung
- 5: Druckschalter für Auswerfvorrichtung
- 6: Führungsnut für Führungszapfen mit Codelesevorrichtung
- 7, 7': Testelement mit Randprofil
- 8: Aussparung
- 9: Testfeld (Detektionsstelle)
- 10: Probenaufgabestelle
- 11: Vorratsbehältnisgehäuse
- 12: Klappe
- 13: Führungszapfen mit Codierung
- 14: Abstandhalter
- 15: Feder
- 16: Platte
- 17: Führungsstange
- 18: abnehmbarer Boden
- 19: Clickverschluß
- 20: Aussparung für Clickverschluß

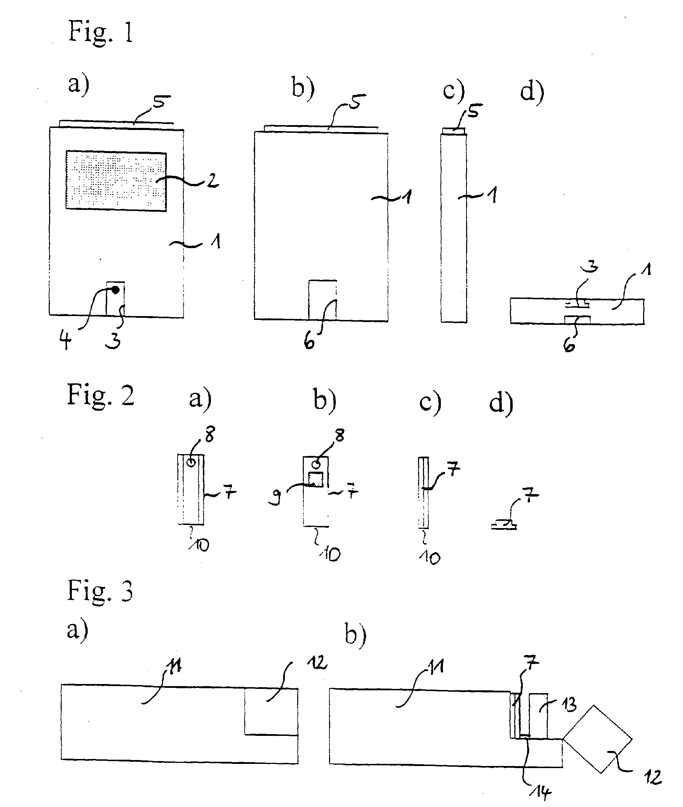

In Figur 1 ist das zum erfindungsgemäßen System gehörige Meßgerät abgebildet. auf der Frontseite des Geräts (Fig. 1a) befindet sich neben einem leicht abzulesenden Display (2) in das Meßgerätegehäuse (1) integriert eine Führungsnut (3) für Testelemente. Aus Fig. 1d) wird ersichtlich, daß die Führungsnut (3) eine Profilierung aufweist, die komplementär zu der Profilierung der Testelemente ist. Im Bereich der Führungsnut (3) befindet sich die Haltevorrichtung (4) für Testelemente. Auf der Rückseite des Meßgerätes (Fig. 1b) befindet sich die Führungsnut (6) für den Führungszapfen des Testelementevorratsbehältnisses. Wie insbesondere aus Fig. 1d) ersichtlich liegt die Führungsnut (3) für Testelemente der Führungsnut (6) für den Führungszapfen genau gegenüber. Die beiden Führungsnuten (3, 6) unterscheiden sich in Form und Dimension, um eine sichere, verwechslungsfreie Handhabung des Systems zu gewährleisten.

In die Führungsnut (6) ist eine Vorrichtung zur automatischen Erfassung der Codierung, die sich auf oder im Führungszapfen des Testelementevorratsbehältnisses befindet, enthalten. Beispielsweise kann es sich dabei um eine Strichcode-Lesevorrichtung handeln.

Das Meßgerät des erfindungsgemäßen Systems verfügt über eine Auswurfvorrichtung für gebrauchte Testelemente. Diese wird durch einen Druckschalter (5) bedient. Mit ihr lassen sich gebrauchte Testelemente aus der Haltevorrichtung (4) lösen und abwerfen.

Das in Fig. 2a bis 2d dargestellte Testelement (7) enthält ein Randprofil (Fig. 2 d), welches zum einen für die richtige Orientierung des Testelements (7) im Meßgerät sorgt und zum anderen in Verbindung mit der Aussparung (8) und der Haltevorrichtung (4) des Meßgeräts sowie dessen Führungsnut (3) eine stabile Fixierung des Testelements (7) im Meßgerät während der Messung bewirkt.

Das Testelement (7) enthält Mittel, beispielsweise einen kapillaraktiven Kanal, die für den Probentransport von der Probenaufgabestelle (10) zur Detektionsstelle (9) sorgen. Die Detektionsstelle (9) ist für optisch auszuwertende Testelemente auf der gerätezugewandten Rückseite des Testelements sichtbar (Fig. 2b). Bei elektrochemischen Sensoren kann die Probenaufgabestelle (10) mit der Detektionsstelle (9) identisch sein.

Fig. 3 stellt schematisch das Testelementevorratsbehäitnis dar. In geschlossenem Zustand (Fig. 3 a) ist nur das Vorratsbehältnisgehäuse (11) und die verschließende Kappe (12) sichtbar. Im offenen Zustand (Fig. 3 b) ist die Klappe (12) zurückgeklappt. Dadurch liegen das erste Testelement (7) und der Führungszapfen mit Codierung (13) frei, die durch einen Abstandshalter (14) einen Spalt zwischen sich frei lassen, in den das Meßgerät zur Entnahme der Testelemente (7) eingeführt werden kann.

Im Vorratsbehältnis liegen neben dem sichtbaren Testelement (7), das sich in der Entnahmeposition befindet, nebeneinandergereiht weitere Testelemente vor, die durch eine Transportvorrichtung, wie z.B. einen Federmechanismus, sukzessive an die Entnahmeposition befördert werden.

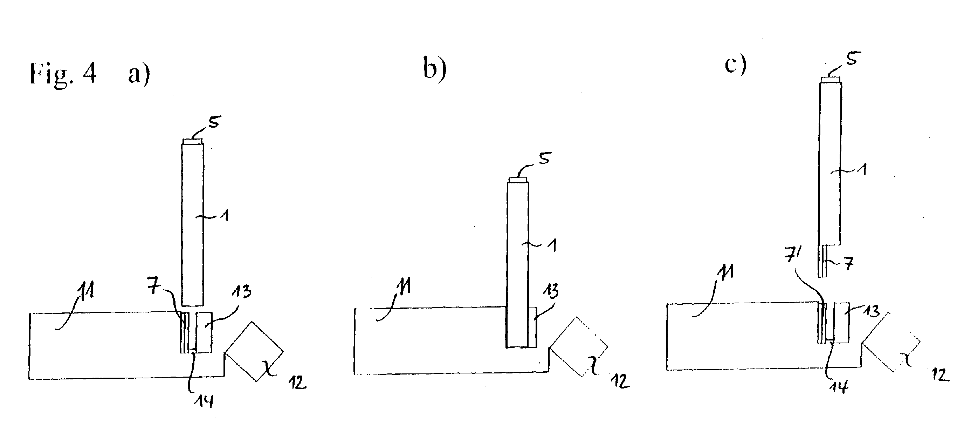

In Fig. 4 ist anhand von drei unterschiedlichen Positionen (Fig. 4a bis c) der Entnahmevorgang für Testelemente (7) aus dem Vorratsbehältnis mit Hilfe des Meßgerätes schematisch dargestellt.

In Fig. 4a ist dargestellt, wie das Meßgerät an das geöffnete Vorratsbehältnis in richtiger gegenseitiger Orientierung herangeführt wird. Die Führungsnut (3) für Testelemente (7) ist dem Testelement, die Führungsnut (6) für den Führungszapfen (13) ist dem Führungszapfen (13) zugewandt.

Das Meßgerät wird bis zum Erreichen der in Fig. 4b gezeigten Position auf das Vorratsbehältnis aufgesetzt. Während des Aufsetzens wird das Testelement (7) positioniert und fixiert und gleichzeitig das Gerät automatisch eingeschaltet.

Beim Abnehmen des Meßgerätes vom Vorratsbehältnis wird der Code auf dem Führungszapfen (13) von der Codelesevorrichtung in der entsprechenden Führungsnut (6) ausgelesen. Das Testelement (7) wird dabei aus dem Vorratsbehältnis entnommen und steht für die Messung zur Verfügung (Fig. 4c).

Durch die Entnahme des Testelements (7) aus dem Vorratsbehältnis wird die Entnahmeposition frei. Diese wird automatisch durch das nachfolgende Testelement (7') besetzt, welches vom Transportmechanismus nachgeführt wird.

Das sich im Meßgerät befindliche Testelement (7) wird mit der Probenaufgabestelle (10) mit der Probe in Kontakt gebracht. Die Probe wird beispielsweise durch Kapillarkräfte zur Detektionsstelle (9) transportiert, wo nach oder bereits während der Detektionsreaktion eine Messung, beispielsweise eine reflexionsphotometrische Beobachtung einer Farbveränderung bedingt durch die Art und die Menge des in der Probenflüssigkeit enthaltenen Analyten, stattfindet. Das direkte Meßergebnis wird vom Meßgerät über geeignete Algorithmen in einen Anzeigewert umgewandelt, angezeigt und gegebenenfalls gespeichert.

Im Anschluß an die Messung wird das Testelement (7) durch betätigen des Druckschalters (5) der Auswurfvorrichtung aus dem Gerät entfernt. Durch die Betätigung des Druckschalters (5) der Auswerfvorrichtung kann das Gerät gleichzeitig abgeschaltet werden.

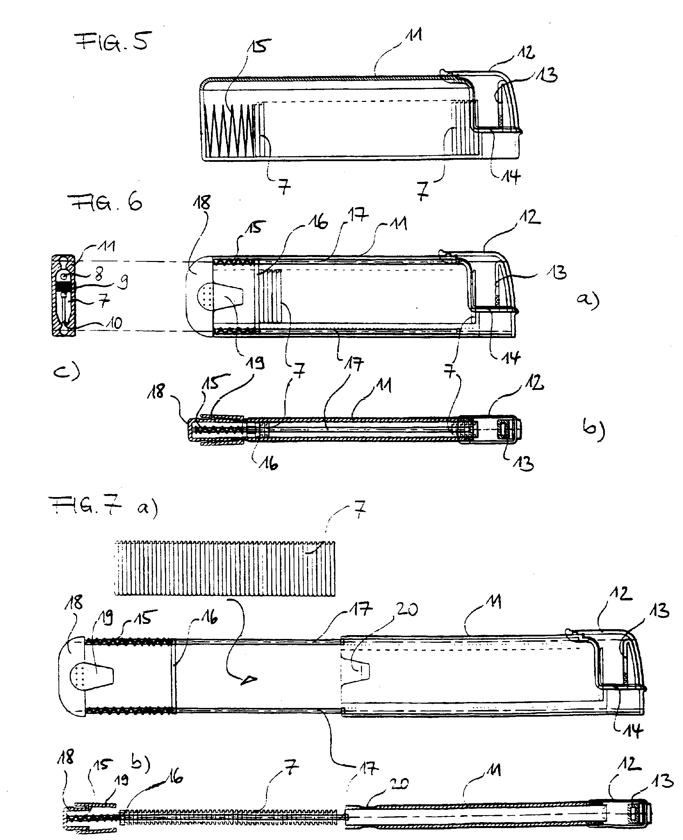

In Figur 5 ist eine besonders bevorzugte Ausführungsform des Testelementevorratsbehälters des erfindungsgemäßen Systems im Längsschnitt von der Seite zu sehen. Durch die Schnittansicht wird der Transportmechanismus sichtbar, der die Testelemente (7) in die Entnahmeposition bringt. Der Transportmechanismus der hier dargestellten Ausfiihrungsform besteht im wesentlichen aus einer Feder (15), die direkt gegen das nächstliegende Testelement (7) drückt. Dadurch werden die Testelemente (7) gegen den Abstandhalter (14) gedrückt, der das in der Entnahmeposition unter der Klappe (12) befindliche Testelement (7) vom Führungszapfen (13) räumlich trennt. Die Führung der Testelemente (7) übernimmt in dieser Ausführungsform im wesentlichen das Vorratsbehältnisgehäuse (11), das entsprechend geometrisch geformt ist.

Figur 6 zeigt einen Längsschnitt von der Seite (a) und von oben (b) sowie einen Querschnitt (c) durch eine weitere besonders bevorzugte Ausführungsform des Testelementevorratsbehälters des erfindungsgemäßen Systems in geschlossenem Zustand. Der Transportmechanismus ist in dieser Ausführungsform durch zwei Federn (15) realisiert, die auf eine Platte (16) drücken, welche wiederum auf die Testelemente (7) einwirken. Die Führung erfolgt hier durch zwei Führungsstangen (17) in Kombination mit der Geometrie des Behältnisgehäuses (11), wie insbesondere aus dem Querschnitt (c) ersichtlich ist.

Die in Figur 6 dargestellte Variante besitzt einen abnehmbaren Boden (18), welcher zum Zwecke des Nachfüllens von Testelementen (7) vom Vorratsbehältnisgehäuse (11) nach Lösen eines Clickverschlusses (19) getrennt werden kann. Figur 7 zeigt einen Längsschnitt von der Seite (a) und von oben (b) durch die besonders bevorzugte Ausführungsform des Testelementevorratsbehälters des erfindungsgemäßen Systems gemäß Figur 6 in zum Beladen mit Testelementen (7) geöffnetem Zustand. Die Testelemente (7) sind mittels eines leicht zu lösenden Klebstoffes über eine kleine Kontaktstelle miteinander verklebt, so daß ein einfaches Befüllen des Testelementevorratsbehältnisses möglich ist.

Zum Befüllen ist der Boden (18) nach dem Lösen des Clickverschlusses (19) abgenommen. Die Führungsstangen (17) halten den Boden (18) in einer geometrisch definierten Position zum Behältnisgehäuse (11). Die Testelemente (7) werden direkt in das Behältnis eingeschoben (Pfeil), welches anschließend durch den Boden (18) wieder verschlossen wird. Dazu rasten die Clickverschlüsse (19) in die dafür vorgesehen Aussparungen (20) im Gehäuse (11) ein. Eine Abdichtung des Gehäuses erfolgt wie bei der Entnahmeklappe (12) durch Dichtlippen oder O-Ringe. Beim Schließen werden die Federn (15) automatisch wieder gespannt und der Transportmechanismus reaktiviert.

## Patentansprüche

1. System zur Analyse von Probenflüssigkeiten beinhaltend
a) Testelemente mit Randprofil
b) ein feuchtigkeitsundurchlässiges, dicht abschließbares Vorratsbehältnis für mindestens 2 Testelemente enthaltend ein Führungselement, einen Abstandshalter zwischen Führungselement und dem als ersten zu entnehmenden Testelement und eine Transportvorrichtung für Testelemente
c) ein stromnetzunabhängiges Meßgerät enthaltend eine Führungsnut für Testelemente, eine Führungsnut für das Führungselement des Testelementevorratsbehältnisses sowie eine Haltevorrichtung für Testelemente,
wobei zur Entnahme eines Testelements das Führungselement in die Führungsnut für das Führungselement und das Testelement in die Führungsnut für Testelemente eingeführt werden.

2. System gemäß Anspruch 1, **dadurch gekennzeichnet, daß** das Vorratsbehältnis feuchtigkeitsundurchlässig und dicht abschließbar ist.

3. System gemäß einem der Ansprüche 1 oder 2, **dadurch gekennzeichnet, daß** das Vorratsbehältnis einen Abstandshalter zwischen Führungselement und dem als ersten zu entnehmenden Testelement enthält.

4. System gemäß einem der Ansprüche 1 bis 3, **dadurch gekennzeichnet, daß** das Meßgerät stromnetzunabhängig und mit einer Hand haltbar ist.

5. System gemäß einem der Ansprüche 1 bis 4, **dadurch gekennzeichnet, daß** die Führungsnut für Testelemente der Führungsnut für das Führungselement des Testelementevorratsbehältnisses gegenüberliegt

6. System gemäß einem der Ansprüche 1 bis 5, **dadurch gekennzeichnet daß** das Meßgerät eine Auswerfvorrichtung für Testelemente enthält.

7. System gemäß einem der Ansprüche 1 bis 6, **dadurch gekennzeichnet, daß** das Führungselement des Testelementevorratsbehältnisses eine Codierung enthält.

8. System gemäß Anspruch 7, **dadurch gekennzeichnet, daß** das Meßgerät eine Vorrichtung zur automatischen Erfassung der Codierung, die auf oder in dem Führungselement des Testelementevorratsbehältnisses enthalten ist, aufweist.

9. System gemäß einem der Ansprüche 1 bis 8, **dadurch gekennzeichnet, daß** die Testelemente Mittel zum Transport der Probe von der Probenaufgabestelle zur Detektionsstelle enthalten.

10. System gemäß Anspruch 9, **dadurch gekennzeichnet, daß** als Mittel zum Transport ein kapillaraktiver Kanal enthalten ist.

11. System gemäß einem der Ansprüche 1 bis 10, **dadurch gekennzeichnet, daß** die Testelemente eine Aussparung aufweisen, durch die sie im Meßgerät gehalten werden können.

12. System gemäß einem der Ansprüche 1 bis 11, **dadurch gekennzeichnet, daß** das Vorratsbehältnis als Transportvorrichtung für Testelemente einen Federmechanismus enthält.

13. System gemäß einem der Ansprüche 1 bis 12, **dadurch gekennzeichnet, daß** das Vorratsbehältnis einen abnehmbaren Boden aufweist.

14. System gemäß einem der Ansprüche 7 bis 13, **dadurch gekennzeichnet, daß** das Führungselement des Vorratsbehältnisses als Codierung einen Strichcode oder einen ROM-Baustein enthält.

15. System gemäß einem der Ansprüche 1 bis 14, **dadurch gekennzeichnet, daß** das Meßgerät als Haltevorrichtung einen Rastmechanismus enthält.

16. System gemäß einem der Ansprüche 8 bis 15, **dadurch gekennzeichnet, daß** das Meßgerät als Vorrichtung zur automatischen Erfassung der Codierung, die auf oder in dem Führungselement des Testelementevorratsbehältnisses enthalten ist, eine Strichcode- oder ROM-Lesevorrichtung enthält.

17. System gemäß einem der Ansprüche 1 bis 16, **dadurch gekennzeichnet, daß** im Meßgerät die Führungsnut für Testelemente und die Führungsnut für das Führungselement des Testelementevorratsbehältnisses unterschiedlich dimensioniert sind.

18. Verfahren zur Bestimmung eines Analyten in einer Probenflüssigkeit mit Hilfe eines Systems gemäß einem der Ansprüche 1 bis 17, wobei
a) aus dem geöffneten Vorratsbehältnis mit Hilfe des Meßgeräts ein Testelement entnommen wird;
b) die Probenaufgabezone des sich im Gerät befindlichen Testelements mit einer Probenflüssigkeit in Kontakt gebracht wird, welche selbständig in die Detektionszone transportiert wird, und dort mit den Komponenten des Testfeldes eine charakteristische, detektierbare Reaktion eingeht;
c) das Ergebnis der Reaktion mit Hilfe des Gerätes erfaßt und angezeigt wird;
d) nach der Analyse das benutzte Testelement aus dem Meßgerät entfernt wird.

## Claims

1. System for the analysis of sample liquids comprising
a) test elements with an edge profile
b) a moisture-impermeable, tightly sealable storage container for at least two test elements containing a guide element, a spacer between the guide element and the first test element to be withdrawn, and a transport device for test elements
c) a measuring instrument that is independent of the power supply network containing a guide groove for test elements, a guide groove for the guide element of the test element storage container as well as a holding device for test elements, wherein the guide element is inserted into the guide groove for the guide element and the test element is inserted into the guide groove for test elements in order to withdraw a test element.

2. System according to claim 1, **characterized in that** the storage container is impermeable to moisture and can be tightly sealed.

3. System according to one of the claims 1 or 2, **characterized in that** the storage container contains a spacer between the guide element and the first test element to be withdrawn.

4. System according to one of the claims 1 to 3, **characterized in that** the measuring instrument is independent of the power supply network and can be held with one hand.

5. System according to one of the claims 1 to 4, **characterized in that** the guide groove for test elements is opposite to the guide groove for the guide element of the test element storage container.

6. System according to one of the claims 1 to 5, **characterized in that** the measuring instrument contains an ejection device for test elements.

7. System according to one of the claims 1 to 6, **characterized in that** the guide element of the test element storage container contains a coding.

8. System according to claim 7, **characterized in that** the measuring instrument has a device for automatically registering the coding that is located on or in the guide element of the test element storage container.

9. System according to one of the claims 1 to 8, **characterized in that** the test elements contain a means to transport the sample from the sample application site to the detection site.

10. System according to claim 9, **characterized in that** it contains a capillary active channel as the transport means.

11. System according to one of the claims 1 to 10, **characterized in that** the test elements have a recess by which means they can be held in the measuring instrument.

12. System according to one of the claims 1 to 11, **characterized in that** the storage container contains a spring mechanism as a transport device for test elements.

13. System according to one of the claims 1 to 12, **characterized in that** the storage container has a removable bottom.

14. System according to one of the claims 7 to 13, **characterized in that** the guide element of the storage container contains a barcode or a ROM component as the coding.

15. System according to one of the claims 1 to 14, **characterized in that** the measuring instrument contains a locking mechanism as the holding device.

16. System according to one of the claims 8 to 15, **characterized in that** the measuring instrument contains a barcode or ROM reading device as the device for automatically registering the coding which is located on or in the guide element of the test element storage container.

17. System according to one of the claims 1 to 16, **characterized in that** the guide groove for test elements and the guide groove for the guide element of the test element storage container have different dimensions in the measuring instrument.

18. Method for the determination of an analyte in a sample liquid with the aid of a system according to one of the claims 1 to 17, **characterized in that**
a) a test element is removed from the opened storage container with the aid of the measuring instrument,
b) the sample application zone of the test element located in the instrument is contacted with a sample liquid which is automatically transported into the detection zone and reacts there characteristically and detectably with the components of the test field,
c) the result of the reaction is detected and displayed with the aid of the instrument,
d) the used test element is removed from the measuring instrument after the analysis.

## Revendications

1. Système pour l'analyse de liquides échantillons, contenant
a) des éléments d'essai comportant un profil marginal ;
b) un récipient de réserve imperméable à l'humidité, qui peut être fermé de manière étanche, pour au moins deux éléments d'essai, contenant un élément de guidage, un écarteur entre l'élément de guidage et l'élément d'essai qui doit être prélevé en premier lieu et un dispositif de transport pour des éléments d'essai ;
c) un appareil de mesure à alimentation autonome contenant une rainure de guidage pour des éléments d'essai, une rainure de guidage pour l'élément de guidage du récipient de réserve des éléments d'essai, ainsi qu'un dispositif de maintien pour des éléments d'essai, l'élément de guidage venant s'insérer dans la rainure de guidage pour l'élément de guidage et l'élément d'essai venant s'insérer dans la rainure de guidage pour des éléments d'essai, à des fins de prélèvement d'un élément d'essai.

2. Système selon la revendication 1, **caractérisé en ce que** le récipient de réserve est imperméable à l'humidité et peut être fermé de manière étanche.

3. Système selon l'une quelconque des revendications 1 ou 2, **caractérisé en ce que** le récipient de réserve contient un écarteur entre l'élément de guidage et l'élément d'essais qui doit être prélevé en premier lieu.

4. Système selon l'une quelconque des revendications 1 à 3, **caractérisé en ce que** l'appareil de mesure possède une alimentation autonome et peut être manipulé à l'aide d'une seule main.

5. Système selon l'une quelconque des revendications 1 à 4, **caractérisé en ce que** la rainure de guidage pour des éléments d'essai est opposée à la rainure de guidage pour l'élément de guidage du récipient de réserve des éléments d'essai.

6. Système selon l'une quelconque des revendications 1 à 5, **caractérisé en ce que** l'appareil de mesure contient un dispositif d'éjection pour des éléments d'essai.

7. Système selon l'une quelconque des revendications 1 à 6, **caractérisé en ce que** l'élément de guidage du récipient de réserve des éléments d'essai contient un codage.

8. Système selon la revendication 7, **caractérisé en ce que** l'appareil de mesure présente un dispositif pour l'enregistrement automatique du codage qui est contenu sur ou dans l'élément de guidage du récipient de réserve des éléments d'essai.

9. Système selon l'une quelconque des revendications 1 à 8, **caractérisé en ce que** les éléments d'essai contiennent des moyens pour le transport de l'échantillon de l'endroit d'application de l'échantillon à l'endroit de détection.

10. Système selon la revendication 9, **caractérisé en ce qu'**il contient un canal à activité capillaire, à titre de moyen de transport.

11. Système selon l'une quelconque des revendications 1 à 10, **caractérisé en ce que** les éléments d'essai présentent un évidement qui permet de les maintenir dans l'appareil de mesure.

12. Système selon l'une quelconque des revendications 1 à 11, **caractérisé en ce que** le récipient de réserve contient un mécanisme à ressort à titre de dispositif de transport pour des éléments d'essai.

13. Système selon l'une quelconque des revendications 1 à 12, **caractérisé en ce que** le récipient de réserve présente un fond amovible.

14. Système selon l'une quelconque des revendications 7 à 13, **caractérisé en ce que** l'élément de guidage du récipient de réserve contient, à titre de codage, un code-barres ou un élément constitutif à mémoire morte.

15. Système selon l'une quelconque des revendications 1 à 14, **caractérisé en ce que** l'appareil de mesure contient un mécanisme d'encliquetage à titre de dispositif de maintien.

16. Système selon l'une quelconque des revendications 8 à 15, **caractérisé en ce que** l'appareil de mesure contient, à titre de dispositif pour l'enregistrement automatique du codage, qui est contenu sur ou dans l'élément de guidage du récipient de réserve des éléments d'essai, un dispositif de lecture de code-barres ou un dispositif de lecture de mémoire morte.

17. Système selon l'une quelconque des revendications 1 à 16, **caractérisé en ce que**, dans un appareil de mesure, la rainure de guidage pour les éléments d'essai et la rainure de guidage pour l'élément de guidage du récipient de réserve des éléments d'essai possèdent des dimensions différentes.

18. Procédé pour la détermination d'un analyte dans un liquide échantillon à l'aide d'un système selon l'une quelconque d es revendications 1 à 1 7, dans lequel
a) un élément d'essai est prélevé du récipient de réserve ouvert, à l'aide de l'appareil de mesure ;
b) la zone d'application de l'échantillon de l'élément d'essai se trouvant dans l'appareil est mise en contact avec un liquide échantillon qui fait l'objet d'un transport autonome jusque dans la zone de détection dans laquelle a lieu une réaction détectable caractéristique avec les composants du champ d'essai ;
c) le résultat de la réaction est enregistré et affiché à l'aide de l'appareil ;
d) après l'analyse, l'élément d'essai utilisé est retiré de l'appareil de mesure.
